# EUROPÄISCHE PATENTSCHRIFT

(11) **EP 1 105 055 B1**
(45) Veröffentlichungstag und Bekanntmachung des Hinweises auf die Patenterteilung: **29.12.2004**
(21) Anmeldenummer: 99923413.1
(22) Anmeldetag: 13.04.1999
(51) Int. Cl.: A61B 17/32

(54) **RESEKTIONSINSTRUMENT**
RESECTION INSTRUMENT
INSTRUMENT DE RESECTION

(30) Priorität: 19.08.1998 DE 29814889 U
(43) Veröffentlichungstag der Anmeldung: 13.06.2001
(73) Patentinhaber: Burgard, Gunther, 66424 Homburg (DE)
(72) Erfinder: Burgard, Gunther, 66424 Homburg (DE)
(74) Vertreter: Grünecker, Kinkeldey, Stockmair & Schwanhäusser Anwaltssozietät
(86) Internationale Anmeldenummer: PCT/EP1999/002484
(87) Internationale Veröffentlichungsnummer: WO 2000/010470

(56) Entgegenhaltungen:
- WO-A-92/15253
- WO-A-96/11638
- DE-U- 29 803 143
- FR-A- 2 588 748
- US-A- 4 832 683

## Beschreibung

Die Erfindung bezieht sich auf ein Resektionsinstrument, insbesondere zum minimalinvasiven, subanodermalen, submucösen Entfernen von Hämorrhoidalgewebe, mit einer schmalen, länglichen Trägervorrichtung, welche an einem vorderen Teil mit einem Resektionsabschnitt zum Entfernen von Gewebe versehen ist, wobei der Resektionsabschnitt eine konvex gewölbte, im Längsschnitt löffelförmige, muldenartige Ausbauchung mit einer Höhlung aufweist.

Aus der FR-A-2 588 748 ist ein Resektionsinstrument dieser Gattung für Herzoperationen bekannt, insbesondere zum Entfernen von Thromben von der Wand der Herzvorkammer. Zum Abtragen der Thromben ist das Frontende der Trägervorrichtung mit einer schneidenartigen Klinge versehen. In der Höhlung ist eine Saugöffnung vorgesehen, die mit einer Unterdruckpumpe verbunden ist. Nach dem Ablösen eines Thrombus wird der Unterdruck eingeschaltet, so dass der Thrombus an die Öffnung gesaugt wird. Anschließend wird das Instrument mit dem angesaugten Thrombus durch die Operationsöffnung geführt und kann dann durch Unterbrechen der Saugwirkung von dem Instrument gelöst werden. Danach kann das Instrument wieder zum Ablösen weiterer Thromben in den Körper eingeführt werden.

Die WO 96/11638 offenbart Resektionseinrichtungen zum Entfernen von Gewebe im Uterus. In dem in den Figuren 2 und 3 dargestellten Ausführungsbeispiel hat eine längliche, etwa rohrförmige Tragervorrichtung an einem vorderen Teil einen Resektionsabschnitt zum Entfernen von Gewebe. Die Trägervorrichtung ist an dem vorderen Ende bereichsweise angeschnitten ausgebildet, so dass sich ein länglicher Schlitz mit im Längsschnitt konvex gewölbter, muldenartiger Ausbauchung ergibt. In der Trägervorrichtung ist ein Fräserkopf mit mehreren Schneiden längsverschieblich angeordnet und tritt im vorderen Ende durch die schlitzförmige Öffnung ragend hervor. Beim Gebrauch wird das vordere Ende der Einrichtung mit dem die Öffnung aufweisenden abgeschrägten Ende zur Gewebewand des Uterus gerichtet. Beim Vorschieben des Fräsers tritt dieser aus dem abgeschrägten Ende hervor und trägt das Gewebe der Uteruswand ab.

In den Figuren 9 und 10A ist ein Ausführungsbeispiel beschrieben, bei welchem an dem Frontende der länglichen Trägervorrichtung ein etwa bogenförmiges elektrochirurgisches Element in Form eines Drahtes angeordnet ist, um Gewebsstreifen vom Uterus abzulösen. Am vorderen Ende der Trägervorrichtung ist ein elektrisch leitender Bereich mit einer Kappe angeordnet, mit welchem Gewebe koaguliert beziehungsweise verschmolzen wird. In gerader Verlängerung der Trägervorrichtung ist im Bereich einer Öffnung mit Kanten ein Fräserelement vorgesehen. Gewebestreifen, die mit dem elektrochirurgischen Bereich abgelöst wurden, werden zu der Fräseröffnung geleitet und dort zerkleinert und abgeführt.

Resektionsinstrumente werden z.B. beim minimalinvasiven, subanodermalen, submucösen Entfernen von Hämorrhoidalgewebe verwendet. Dabei wird das schmale, längliche Resektionsinstrument an der Stelle zwischen dem Anoderm und der Haut der Pobacke eingeführt, so daß das empfindliche Anorderm kaum verletzt wird. Das Hämorrhoidalgewebe wird unterhalb des Anorderms mit Hilfe von Ultraschall präpariert und fragmentiert und erst danach mit dem Resektionsinstrument reseziert.

Bei einem aus DE-U-298 03 143 bekannten Resektionsinstrument der oben genannten Gattung ist an einem Frontende einer Trägervorrichtung eine gewebeabtragende Fräsereinheit zum Resezieren vorgesehen. Die Fräser erstrecken sich durch die Trägervorrichtung und befinden sich etwa im Bereich von Fräseröffnungen, welche im Mantelbereich über den Umfang der Trägervorrichtung verteilt vorgesehen sind. Dieses Resektionsinstrument hat sich prinzipiell bewährt. Beim Einsatz kann unter der gebotenen Vorsicht und Übung in der Handhabbarkeit das Gewebe lokal präzise reseziert werden.

Der Erfindung liegt die Aufgabe zugrunde, das Resektionsinstrument der eingangs genannten Gattung dahingehend zu verbessern, daß damit Gewebe bei möglichst einfacher Handhabung des Instruments präzise und möglichst schonend reseziert werden kann.

Diese Aufgabe wird erfindungsgemäß gelöst mit einem Resektionsinstrument mit den Merkmalen des charakterisiesenden Teils des Anspruchs 1.

Durch die gewölbte löffelförmige, muldenartige Ausbauchung ist eine Art Höhlung geschaffen, in der die Resektion stattfindet. Das Instrument kann so gesteuert werden, daß nur die zu entfemenden Gewebeteile in den Bereich der Höhlung und an die Resektionsfläche gelangen. Die muldenförmige Ausbauchung bildet eine Art Schutz, so daß umliegende Gewebeteile nicht beeinträchtigt werden. An der der Innenseite abgewandten Rückseite der Ausbuchtung ist das Gewebe geschützt. Damit kann man mit der Rückseite präzise an Gewebeteilen entlangfahren und auf der Innenseite des Instruments krankes Gewebe gezielt behandeln.

Die muldenartige Ausbuchtung kann auch sphärisch ausgebildet sein.

Mit dem erfindungsgemäß ausgebildeten Resektionsabschnitt läßt sich Gewebe sehr präzise resezieren, wobei benachbarte gesunde Gewebeteile kaum in Mitleidenschaft gezogen werden. Die Handhabung des Gerätes ist denkbar einfach.

Bei der Verwendung als Hämorrhoidenresektionsinstrument wird das Instrument an einer kleinen Inzision im Grenzbereich zwischen dem Anoderm, das ist die Übergangshaut am Ende des Analkanals zwischen der Schleimhaut des Mastdarms und der außenliegenden Haut der Pobacke, eingeführt und rückseitig mit der Ausbauchung anliegend an dem Anoderm vorgeschoben. An der an der Innenseite der muldenartigen Ausbauchung anliegenden Resektionsfläche wird mit Hilfe der Resektionseinrichtung Hämorrhoidalgewebe entfernt. In der Regel wurde das Hämorrhoidalgewebe vorher mit einer vibrationschirurgischen Einheit präpariert und fragmentiert.

Über die Saugöffnung wird das zu resezierende Gewebe in die Ausbauchung zum Resektionsabschnitt hin gesaugt, so dass es reseziert werden kann. Dies betrifft insbesondere lose, zum Beispiel vorfragmentierte Gewebeteile. Wichtige gesunde Gewebsteile, wie zum Beispiel Muskeln, insbesondere im angespannten Zustand, werden nicht angesaugt und bleiben daher beabstandet von der Ausbauchung und werden nicht in die Höhlung eingezogen.

Vorzugsweise kann die Trägervorrichtung einen etwa stabförmigen, sich in einer Längsrichtung erstreckenden Halteabschnitt aufweisen, und die muldenartige Ausbauchung von der Längsrichtung abweichend, sich konvex mindestens ansatzweise auf die Längsrichtung zurückkrümmend ausgebildet sein. Der sich in Längsrichtung erstreckende Halteabschnitt gibt die Orientierung für die Ausrichtung des Resektionsabschnitts. Von der Längsrichtung abweichend ist dann die Resektionsfläche etwas zurückversetzt in der muldenartigen Ausbauchung vorgesehen, so daß eine Lokalisierung der Ausbauchung von außen möglich ist. Durch die Rückkrümmung der Ausbauchung auf die Längsrichtung ergibt sich die im Längsquerschnitt löffelartige Form, die von der Handhabung so gesteuert werden kann, daß nur zu entfernendes Gewebe in den Bereich der Resektionsfläche gelangt.

Die muldenartige Ausbauchung muß sich nicht unbedingt bis auf die Längsrichtung zurückkrümmen. Obwohl diese Ausbildung möglich ist, kann das Ende der Ausbauchung auch beabstandet von der Längsrichtung enden. Unter Längsrichtung ist vorzugsweise eine Gerade zu verstehen. Denkbar ist aber auch z.B. eine leicht bogenförmige Ausbildung des Halteabschnitts. Dabei kann der Halteabschnitt auch stummelartig kurz ausgebildet sein.

Besonders vorteilhaft kann die Ausbauchung ausgehend von dem Halteabschnitt einen von der Längsrichtung abweichend nach außen gekrümmten ersten Bogen und daran anschließend einen entgegen dem ersten Bogen gekrümmten zweiten Bogen aufweisen, an dessen der Längsrichtung zugewandten Innenseite wenigstens ein Teil der Resektionsfläche angeordnet ist. Durch den ersten Bogen weicht der Halteabschnitt von der Längsrichtung ab. Dem entgegengesetzt ist der zweite Bogen vorgesehen, der den Hauptteil der Ausbauchung bildet und an dessen Innenseite zur Längsrichtung geneigt die Resektionsfläche vorgesehen ist. Die Bögen können wahlweise kreisförmig gewählt sein, wobei ihre Mittelpunkte auf unterschiedlichen Seiten des Instruments angeordnet sind, wodurch die entgegengesetzte Krümmung der Radien entsteht. Die Ausbildung anhand der Bögen ist besonders günstig für sanfte Übergänge längs des Instruments, so daß es störungsfrei durch eine kleine Inzision in eine Körperhöhlung vorgeschoben werden kann.

Günstigerweise kann die Resektionsfläche ausschließlich auf den Bereich des zweiten Bogens begrenzt sein. Damit ist die Resektionsfläche präzise lokalisiert und in ihrem gesamten Bereich geschützt innerhalb der muldenartigen Ausbauchung angeordnet.

Wahlweise kann der Resektionsabschnitt mindestens im Bereich der Ausbauchung breiter sein als der Halteabschnitt. Damit läßt sich der Höhlungsbereich großräumiger gestalten. In einem Körperiumen kann der Resektionsabschnitt großflächiger angewendet werden, während der Halteabschnitt zum Handhaben des Instruments durch eine möglichst kleine Körperöffnung relativ schmal ausgebildet sein kann.

Als Variante der Erfindung kann die Ausbauchung im Querschnitt relativ zu einem Halteabschnitt der Trägervorrichtung abgeflacht ausgebildet sein. Durch die flache Ausbildung läßt sich die Ausbauchung sehr gut in schmale Körperspalten einbringen, wobei benachbartes Gewebe kaum beeinträchtigt wird.

Gemäß einer bevorzugten Ausführungsform kann eine Lichtspendeeinrichtung an der der Innenseite abgewandten Rückseite des Resektionsabschnitts vorgesehen sein. Das von der Lichtspendeeinrichtung ausgesendete Licht kann als Positionierhilfe von dem operierenden Arzt verwendet werden. Das Licht wirkt diaphanoskopisch; es durchleuchtet Gewebsteile zur Rückseite. Der behandelnde Arzt erkennt anhand des Lichts und Schattenrissen des umliegenden Gewebes die Position des Resektionsabschnittes. Die Rückseite ist üblicherweise nahe der Außenoberfläche des Körpers vorgesehen, so daß über die Lichtspendeeinrichtung die Position des Instruments erkennbar ist und das Instrument entsprechend gesteuert werden kann. Insbesondere bei Hämorrhoidenresektionen liegt die Rückseite des Resektionsabschnitts etwa an dem Anoderm an und mit Hilfe des durch das Anoderm scheinende Licht ist die genaue Position des Resektionsabschnitts erkennbar.

Besonders vorteilhaft kann eine Lichtspendeeinrichtung an einem Frontende des Resektionsabschnitts angeordnet sein. Durch diese Anordnung läßt sich der Ort des vorderen Bereichs des Instruments erkennen, so daß der behandelnde Arzt eine Information über den Vorschub des Instruments im Körper erhält.

In besonderer Weise kann mindestens das Frontende des Resektionsabschnitts eine Ultraschalleinheit zum Präparieren und Fragmentieren von Gewebe aufweisen. Damit kann auf vibrationschirurgischem Weg das Gewebe präpariert und fragmentiert werden. Bei einer Hämorrhoidenoperation kann das Hämorrhoidalgewebe vom inneren Schließmuskel auf der einen Seite und vom Anoderm auf der anderen Seite isoliert werden. Dabei bleibt das Anoderm erhalten und der Muskulus Intemus unbeschädigt.

Als ultraschallchirurgisches Werkzeug kann z.B. eine Frequenz von 20.000 bis 40.000 Hertz verwendet werden. Damit kann Hämorrhoidalgewebe ganz gezielt und ohne Beschädigung des gesunden Gewebes präpariert und fragmentiert werden. Danach kann es mit der Resektionseinrichtung zerkleinert und abgetragen werden, so daß es stückweise aus dem Bereich zwischen Schließmuskel und Anoderm entfembar ist.

Gemäß einer besonderen Ausführungsform kann der Resektionsabschnitt als ultraschallschwingende Gewebezerkleinerungseinrichtung zum Präparieren und Fragmentieren von Gewebe ausgebildet sein. Wenn der Resektionsabschnitt selbst als Ultraschalleinheit ausgebildet ist, so schwingt praktisch der gesamte Resektionsabschnitt oder das gesamte Instrument, so daß umliegendes krankes Gewebe präpariert wird, während gesundes Gewebe nicht angegriffen wird.

Möglicherweise kann der Resektionsabschnitt eine Spülöffnung aufweisen, die in einen mit einem Spülsystem verbindbaren Spülkanal mündet. Über die Spülöffnung kann Spülflüssigkeit zugeführt oder wahlweise auch abgeführt werden. Damit kann der zu behandelnde Bereich ausgespült werden und eventuell freie Gewebsteile können gelöst werden. Als Spülflüssigkeit, beziehungsweise -medium kann beispielsweise physiologische Kochsalzlösung beziehungsweise Ringerlaktatlösung über die Öffnung appliziert werden. Dies ist insbesondere bei der Anwendung von Elektrovaporisation (Gewebeverdampfung) als Resektionsmethode günstig. Die Spülflüssigkeit kann entweder durch einen Spülkanal des Instruments wieder zurückfließen oder an der Inzisionsstelle zwischen Haut und Instrument austreten. Zum Rückführen von Spülflüssigkeit kann auch die Saugöffnung verwendet werden. Die Spülflüssigkeit kann auch zum Reinigen der Resektionseinrichtung verwendet werden, z.B. wenn die Resektionsöffnung droht, durch Gewebeteile zu verstopfen. Wahlweise kann die Spülung auch zu Kühlzwecken verwendet werden.

Gemäß einer besonderen Ausgestaltung der Erfindung kann die Spülöffnung mindestens bereichsweise in der Resektionsfläche vorgesehen sein.

Ein Ausführungsbeispiel der Erfindung ist in der Zeichnung dargestellt und wird nachstehend erläutert. Es zeigen:
- Fig. 1: eine Seitenansicht auf einen ersten Teil eines erfindungsgemäßen Resektionsinstruments,
- Fig. 2: eine Unteransicht auf das in Figur 1 dargestellte Resektionsinstrument,
- Fig. 3: eine Draufsicht auf die Rückseite des in Figur 1 dargestellten Resektionsinstruments,
- Fig. 4: eine schematische Skizze einer Handhabe mit anschließbaren Versorgungseinheiten eines erfindungsgemäßen Resektionsinstruments, und
- Fig. 5: eine Prinzipdarstellung eines erfindungsgemäßen Resektionsinstruments im Enddarmbereich beim Entfernen von Hämorrhoidalgewebe.

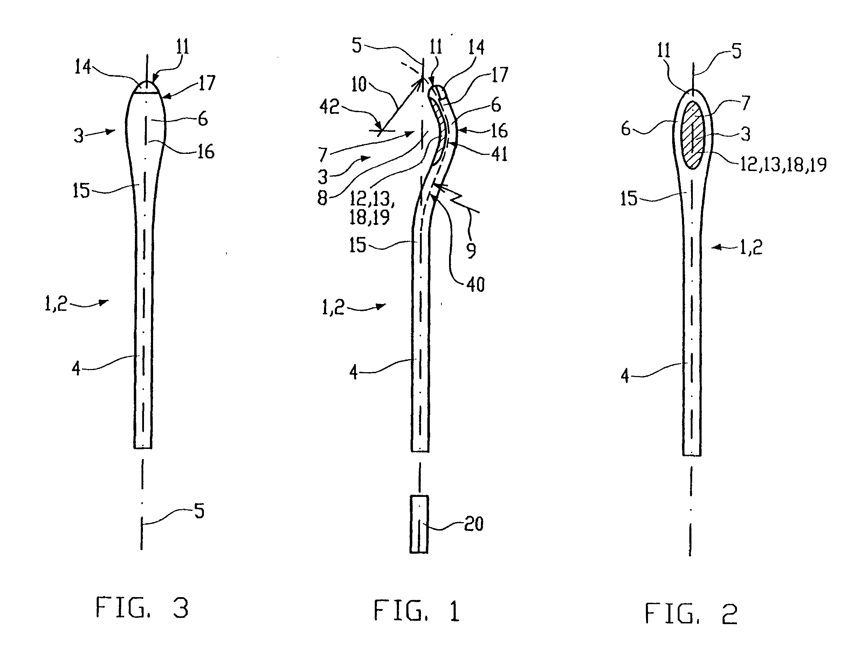

In Figur 1 ist ein erstes Teil 1 eines erfindungsgemäßen Resektionsinstruments 2 in einer Seitenansicht dargestellt. In Figur 2 ist das erste Teil in geklappter Darstellung abgebildet, so daß man gerade auf die Unterseite des Instruments 2 sieht. In Figur 3 ist das in Figur 1 dargestellte Instrument 2 in zur anderen Richtung geklappten Stellung abgebildet, so daß man gerade auf die Rückseite des Instruments 2 sieht.

Der in den Figuren 1 bis 3 dargestellte erste Teil 1 des Resektionsinstruments 2 besteht aus einer schmalen, länglichen Trägervorrichtung 15 mit einem Resektionsabschnitt 3 und einem Halteabschnitt 4. Der Halteabschnitt ist etwa stabförmig ausgebildet und erstreckt sich längs einer als Geraden ausgebildeten Längsrichtung 5. Die Längsrichtung 5 kann in einem anderen Ausführungsbeispiel auch z.B. leicht gebogen sein.

Die Trägervorrichtung 15 ist hohl ausgebildet, so daß sich die für die Resektion notwendigen Einrichtungen durch sie hindurch bis in ihren Wirkbereich im Resektionsabschnitt 3 erstrecken können.

An den Halteabschnitt 4 grenzt der Resektionsabschnitt 3 an, der von der Längsrichtung 5 abweicht und eine in Seitenansicht etwa muldenartige Ausbauchung 6 aufweist. Die Ausbauchung 6 ist etwa konvex gewölbt und hat in einem die Längsrichtung 5 enthaltenen Längsschnitt etwa Löffelform. Die Ausbauchung bildet an der verlängerten Längsrichtung 5 zugewandten Innenseite 7 etwa eine Höhlung 8.

Die Form des Resektionsabschnitts 3 wird durch zwei Bögen 40, 41 zweier Radien gebildet. Der erste Bogen 40 geht von der Längsrichtung 5 abweichend nach außen aus und hat einen ersten Radius 9. Der konvex gewölbte, muldenförmige Teil des Resektionsabschnitts, also die Ausbauchung 6, wird durch den zweiten Bogen 41 eines zweiten Radius 10 gebildet, dessen Mittelpunkt auf einer in Figur 1 dargestellten entgegengesetzten Seite des Resektionsinstruments 2 zu dem Mittelpunkt 42 des ersten Radius 9 angeordnet ist. Dadurch ergibt sich eine doppelt entgegengesetzt gewundene Form des Resektionsabschnitts 3, der aus zwei Bögen 40, 41 mit entgegengesetzten Krümmungen zusammengesetzt ist.

Das vorderste Ende 11 der Ausbauchung 6 ist auf die Längsrichtung 5 zu gekrümmt ausgebildet. Es endet im Abstand von der Längsrichtung 5. Wahlweise kann auch eine Ausführungsform gewählt werden, bei der sich die Ausbauchung 6 oder das vorderste Ende 11 bis zur Längsrichtung 5 zurückerstreckt.

An der Innenseite 7 der Ausbauchung 6 ist eine Resektionsfläche 12 angeordnet. Die Resektionsfläche 12 erstreckt sich ausschließlich in dem von dem zweiten Radius 10 gebildeten Bereich. Sie ist etwa länglich und maulförmig an der Innenseite 7 vorgesehen. Im Bereich der Resektionsfläche 12 ist eine Resektionseinrichtung 13 vorgesehen. Sie kann beispielsweise aus einer Vorrichtung zur Elektrovaporisation, einer Vorrichtung für Hochfrequenztechnik, einem Laser, einer Wasserstrahleinrichtung oder einer mechanischen Resektionseinrichtung, wie ein rotierendes Messer, also eine Fräseinrichtung, bestehen. Vorzugsweise ist die Resektionsfläche 12 als Resektionsöffnung ausgebildet, in der z.B. ein rotierendes Messer als Fräseinrichtung zum Resezieren von Gewebe dient.

Bei Anwendung einer Vorrichtung zur Elektrovaporisation, eine Art Gewebeverdampfung, empfiehlt sich als Spülmedium eine physiologische Kochsalzlösung oder Ringerlaktatlösung. Mit der Elektrovaporisationsvorrichtung kann Gewebe durch bipolare Elektrochirurgie prinzipiell aufgelöst, beziehungsweise verdampft, werden.

Wie in Figur 2 dargestellt, ist der Resektionsabschnitt im Bereich der Ausbauchung 6 breiter als der Halteabschnitt 4. Die Breite des Resektionsabschnitts 3 entspricht ca. 0,75 bis 1 cm. Die Ausbauchung 6 ist relativ zu dem Halteabschnitt 4 im die Radien 9, 10 enthaltenen Querschnitt abgeflacht ausgebildet.

An dem vordersten Ende 11 der Ausbauchung 6 ist eine Lichtspendeeinrichtung 14 vorgesehen. Die Lichtspendeeinrichtung 14 gibt diaphanoskopisch verwertbares Licht ab. Sie ist vorzugsweise als Kaltlichtquelle ausgebildet, z.B. als Glasfaserlichtleiter, der sich durch die Trägervorrichtung 15 hindurch erstreckt und am vordersten Ende 11 Licht nach außen abgibt.

Die Lichtspendeeinrichtung 14 gibt Licht von der der Innenseite 7 der Ausbauchung 6 abgewandten Rückseite 16 ab. Die Lichtspendeeinrichtung 14 ist damit an der zur Resektionsfläche 12 entgegengesetzten Rückseite 16 vorgesehen und strahlt davon ausgehend Licht ab.

Der gesamte Resektionsabschnitt ist als ultraschallschwingende Gewebezerkleinerungseinrichtung zum Präparieren und Fragmentieren von Gewebe ausgebildet. Dies bedeutet, daß der gesamte Resektionsabschnitt bzw. dieser Teil der Trägervorrichtung 15 in Schwingung versetzt werden kann, um das krankhafte Gewebe vorzupräparieren. Wahlweise ist nur das Frontende 11 des Resektionsabschnitts als Ultraschalleinheit ausgebildet oder weist eine integrierte Ultraschalleinrichtung auf. Dabei kann am Frontende 11 eine Wirkfläche der Ultraschalleinheit vorgesehen sein, die das Gewebe beim Vordringen des Instruments präpariert. Für den Ultraschall werden üblicherweise Frequenzen von 20.000 bis 40.00 Hertz verwendet.

Die Resektionsfläche ist als Resektionsöffnung 12 ausgebildet, die gleichzeitig als Saugöffnung 18 dient. Dabei kann sich die Saugöffnung über die gesamte Resektionsfläche 12 oder nur über einen Teil erstrecken. Die Saugöffnung 18 ist an eine Unterdruckquelle anschließbar, die über einen sich in der Trägervorrichtung 15 zur Saugöffnung 18 erstreckenden Saugkanal mit Unterdruck versorgt wird.

Der Resektionsabschnitt weist eine Spülöffnung 19 auf, die in einen mit einem Spülsystem verbindbaren Spülkanal mündet. Der Spülkanal erstreckt sich durch die Trägervorrichtung 15 hindurch. Die Spülöffnung 19 kann sich über die gesamte Resektionsfläche 12 erstrecken oder nur einen Teil davon ausfüllen.

Die Ausbauchung 6 kann auch sphärisch gestaltet sein, so daß sie einer Löffelform sehr nahe kommt und eine dreidimensionale Wölbung bildet.

Das Frontende 11 kann, wie in den Figuren 1 bis 3, ausgerundet ausgebildet sein, oder eckig, eher spatelförmig.

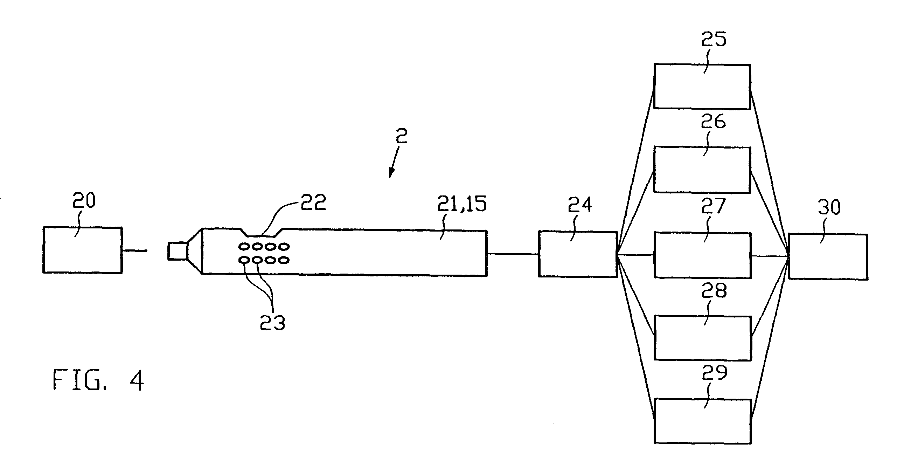

In Figur 1 ist ein Verbindungsglied 20 dargestellt, mit dem das erste Teil 1 mit einem als Handhabe ausgebildeten zweiten Teil 21 verbindbar ist. Das zweite Teil 21 ist in Figur 4 dargestellt.

Die Handhabe 21 weist eine in der Außenseite als Vertiefung eingeformte Markierung 22 auf. Um den Bereich der Markierung 22 sind mehrere Griffnoppen 23 vorgesehen.

Wie schematisch angedeutet, ist das Instrument 2 mit einer Fußschaltung 24 versehen, mit der die Ultraschalleinheit 17 und/oder die Resektionseinrichtung 13 unabhängig voneinander zu- und abschaltbar ist. Eventuell können darüber auch die Saugfunktion und/oder die Spülfunktion geschaltet werden.

Blockartig sind ein Antriebsgerät 25 für die Resektionseinrichtung 13, ein Vibrationsantrieb 26 für die Ultraschalleinheit 17, die Saugpumpe 27, das Spülsystem 28 und eine Lichtquelle 29 dargestellt. Alle diese Geräte können durch einen Mikroprozessor 30 geregelt werden.

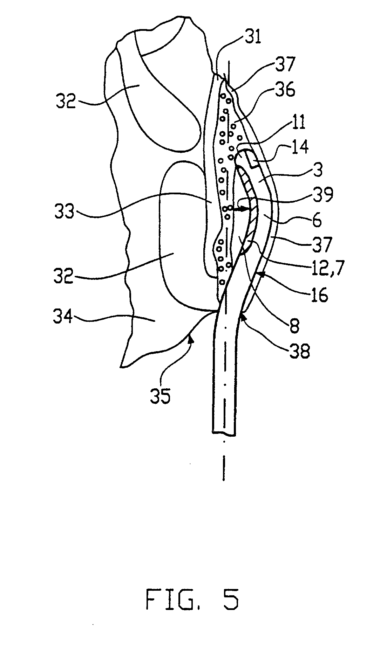

In Figur 5 ist ein Querschnitt durch einen Teil eines menschlichen Enddarmbereichs dargestellt, mit einem Enddarm 31, einem äußeren Schließmuskel 32, einem inneren Schließmuskel 33, Unterhautfettgewebe 34 und äußerer Haut 35 einer Pobacke. Im Bereich der Afteröffnung befindet sich links ein vergrößerter Hämorrhoidalkomplex 36. Das Hämorrhoidalgewebe 36 befindet sich unter dem Anoderm 37, das die Haut zwischen der Haut der Pobacke und der inneren Schleimhaut des Enddarms bildet. Das Anoderm 37 weist eine hohe sensible Nervenversorgung auf und sorgt für die sensorische Kontinenz.

Zwischen dem Anoderm 37 und der Haut 35 der Pobacke ist eine Inzision 38 gemacht, durch die das Resektionsinstrument 2 rückseitig an dem Anoderm 37 anliegend eingeschoben ist. Die Ausbauchung 6 ist mit der Innenseite 7 dem Hämorrhoidalgewebe zugewendet. Die Lichtspendeeinrichtung 14 ist in Richtung auf das Anoderm 37 gerichtet und gibt Licht ab, das durch das Anoderm 37 hindurchscheint.

Bei der gewählten Darstellung ist die Saugpumpe 27 noch nicht aktiv, so daß das Hämorrhoidalgewebe 36 kaum in die Höhlung 8 eintritt und somit beabstandet zur Resektionsfläche 12 angeordnet ist. Mit dem Pfeil 39 ist die Bewegungsrichtung des Hämorrhoidalgewebes 36 angedeutet wenn die Saugpumpe 27 aktiv ist und das Gewebe 36 mit Unterdruck in Richtung auf die Resektionsfläche 12 gezogen wird. Dabei sind die gesunden Gewebsteile wie Muskeln und der daran befestigte Enddarm 31 in der Regel angespannt und folgen der Saugbewegung nicht. Nur krankhaftes Hämorrhoidalgewebe 36 gelangt an die Resektionsfläche 12 und wird dort reseziert.

Im folgenden wird die Wirkungs- und Funktionsweise des in der Zeichnung dargestellten Ausführungsbeispiels eines erfindungsgemäßen Resektionsinstruments bei der Resektion von Hämorrhoiden näher erläutert.

Zum Vorbereiten des Eingriffs wird zunächst die Blutzufuhr durch Ligaturen an einer inneren Schicht des inneren Schließmuskels 33 unterbunden. Anschließend wird im Basisbereich das Hämorrhoidaigewebe nahe der Haut 35 eine kleine Inzision gemacht. Durch die Inzision 38 wird das Resektionsinstrument 2 mit seinem Frontende 11 voraus eingeschoben.

Es wird zunächst nur die vibrationschirurgische Gewebezerkleinerungseinrichtung, das heißt die Ultraschalleinheit 17, benutzt und das Hämorrhoidalgewebe präpariert und fragmentiert. Vorzugsweise wird dabei entlang der Kante des inneren Schließmuskels 32 auf der einen Seite und direkt unterhalb des Anoderms 37 auf der anderen Seite vorgegangen. Der Weg des Frontendes 11 in dem Gewebe wird mit Hilfe der Lichtspendeeinrichtung 12 diaphanoskopisch von außen durch das Anoderm betrachtet und gesteuert.

Nach dem Präparieren und Fragmentieren wird die Resektionseinrichtung 13 eingesetzt und das Hämorrhoidalgewebe zerkleinert und mit Hilfe des Spülsystems oder Saugsystems abgeführt. Dabei wird die Ausbauchung 6 mit ihrer Rückseite 16 an das Anoderm 37 angelegt, so daß die Innenseite 7 der Ausbauchung dem Hämorrhoidalgewebe 36 zugewendet ist. Erst bei Einsatz der Saugpumpe 27 wird das Hämorrhoidalgewebe in die Höhlung 8 eingezogen und gelangt in Kontakt mit der Resektionsfläche 12 und wird mit Hilfe der Resektionseinrichtung 13 entfernt.

Mit dem erfindungsgemäßen Resektionsinstrument 2 ist die als minimal-invasive subanodermale Hämorrhoiddektomie bekannte Operationstechnik durchführbar, die in der DE-U-298 03 143.4 ausführlich beschrieben ist.

## Patentansprüche

1. Resektionsinstrument (2), insbesondere zum minimalinvasiven, subanodermalen, submucösen Entfernen von Hämorrhoidalgewebe (36), mit einer schmalen, länglichen Trägervorrichtung (15), welche an einem vorderen Teil mit einem Resektionsabschnitt (3) zum Entfernen von Gewebe (36) versehen ist, wobei der Resektionsabschnitt (3) eine konvex gewölbte, im Längsschnitt löffelförmige, muldenartige Ausbauchung aufweist,
**dadurch gekennzeichnet,**
**dass** an der Innenseite (7) der muldenartigen Ausbauchung (6) im eine mit einer gewebezerkleinernden Resektionseinrichtung (13) versehene Resektionsfläche (12) angeordnet ist und im Bereich der Resektionsfläche (12) mindestens eine an eine Unterdruckquelle (27) anschließbare Saugöffnung (18) vorgesehen ist.

2. Instrument nach Anspruch 1,
**dadurch gekennzeichnet,**
**daß** die Trägervorrichtung (40) einen etwa stabförmigen, sich in einer Längsrichtung (5) erstreckenden Halteabschnitt (4) aufweist, und die muldenartige Ausbauchung (6) von der Längsrichtung (5) abweichend, sich konvex mindestens ansatzweise auf die Längsrichtung (5) zurückkrümmend ausgebildet ist

3. Instrument nach Anspruch 2,
**dadurch gekennzeichnet,**
**daß** die Ausbauchung (6) ausgehend von dem Halteabschnitt (4) einen von der Längsrichtung (5) abweichenden nach außen gekrümmten ersten Bogen (40) und sich daran anschließend einen entgegen dem ersten Bogen (40) gekrümmten zweiten Bogen (41) aufweist, an dessen der Längsrichtung (5) zugewandten Innenseite (7) wenigstens ein Teil der Resektionsfläche (12) angeordnet ist

4. Instrument nach Anspruch 3,
**dadurch gekennzeichnet,**
**daß** die Resektionsfläche (12) ausschließlich auf den Bereich des zweiten Bogens begrenzt ist.

5. Instrument nach einem der vorhergehenden Ansprüche,
**dadurch gekennzeichnet,**
**daß** der Resektionsabschnitt (3) mindestens im Bereich der Ausbauchung (6) breiter ist als der Halteabschnitt (4).

6. Instrument nach einem der vorhergehenden Ansprüche,
**dadurch gekennzeichnet,**
**daß** die Ausbauchung (6) im Querschnitt relativ zu einem Halteabschnitt (4) der Trägervorrichtung (40) abgeflacht ausgebildet ist

7. Instrument nach einem der vorhergehenden Ansprüche,
**dadurch gekennzeichnet,**
**daß** eine Lichtspendeeinrichtung (14) an der der Innenseite (7) abgewandten Rückseite (16) des Resektionsabschnitts (3) vorgesehen ist.

8. Instrument nach einem der vorhergehenden Ansprüche,
**dadurch gekennzeichnet,**
**daß** eine Lichtspendeeinrichtung (14) an einem Frontende (11) des Resektionsabschnitts (3) angeordnet ist.

9. Instrument nach einem der vorhergehenden Ansprüche,
**dadurch gekennzeichnet,**
**daß** mindestens das Frontende (11) des Resektionsabschnitts (3) eine Ultraschalleinheit (17) zum Präparieren und Fragmentieren von Gewebe (36) aufweist.

10. Instrument nach einem der vorhergehenden Ansprüche,
**dadurch gekennzeichnet,**
**daß** der Resektionsabschnitt (3) als ultraschallschwingende Gewebezerkleinerungseinrichtung (17) zum Präparieren und Fragmentieren von Gewebe (36) ausgebildet ist.

11. Instrument nach einem der vorhergehenden Ansprüche,
**dadurch gekennzeichnet,**
**daß** der Resektionsabschnitt (3) eine Spülöffnung (19) aufweist, die in einen mit einem Spülsystem verbindbaren Spülkanal mündet.

12. Instrument nach Anspruch 11,
**dadurch gekennzeichnet,**
**daß** die Spülöffnung (19) mindestens bereichsweise in der Resektionsfläche (12) vorgesehen ist.

## Claims

1. Resection instrument (2), especially for the minimally invasive, subanodermal, submucuous removal of hemorrhoidal tissue (36), comprising a narrow oblong carrier means (15) provided with a resection portion (3) for removing tissue (36) on a front part, wherein the resection portion (3) has a trough-like bulge which is curved in a convex manner and spoon-shaped in the longitudinal section,
**characterized in that**
a resection surface (12) provided with a tissue-disintegrating resection means (13) is disposed on the inner side (7) of the trough-like bulge (6), and that at least one suction opening (18) to be connected to a negative pressure source (27) is provided in the area of the resection surface (12).

2. Instrument according to claim 1,
**characterized in that**
the carrier means (40) comprises an approximately rod-shaped holding portion (4) extending in a longitudinal direction (5) and the trough-like bulge (6), deviating from the longitudinal direction (5), is designed in a fashion that it at least partly bends back to the longitudinal direction (5) in a convex manner.

3. Instrument according to claim 2,
**characterized in that**
the bulge (6), starting at the holding portion (4), is provided with a first arch (40) curved outwardly and deviating from the longitudinal direction (5) and, joining the same, with a second arch (41) bent in a direction opposite to the first arch (40), at the inner side (7) of which facing the longitudinal direction (5) at least a part of the resection surface (12) is provided.

4. Instrument according to claim 3,
**characterized in that**
the resection surface (12) is exclusively limited to the area of the second arch.

5. Instrument according to one of the preceding claims,
**characterized in that**
the resection portion (3) is broader than the holding portion (4) at least in the area of the bulge (6).

6. Instrument according to one of the preceding claims,
**characterized in that**
the bulge (6) has a flat configuration in the cross-section relative to a holding portion (4) of the carrier means (40).

7. Instrument according to one of the preceding claims,
**characterized in that**
a light-dispensing means (14) is provided on the rear side (16) of the resection portion (3) facing away from the inner side (7).

8. Instrument according to one of the preceding claims,
**characterized in that**
a light-dispensing means (14) is disposed at a front end (11) of the resection portion (3).

9. Instrument according to one of the preceding claims,
**characterized in that**
at least the front end (11) of the resection portion (3) is provided with an ultrasonic unit (17) for dissecting and fragmenting tissue (36).

10. Instrument according to one of the preceding claims,
**characterized in that**
the resection portion (3) is configured as an ultrasonic vibration tissue disintegration device (17) for dissecting and fragmenting tissue (36).

11. Instrument according to one of the preceding claims,
**characterized in that**
the resection portion (3) comprises a rinsing opening (19) joining a rinsing channel
to be connected to a rinsing system.

12. Instrument according to claim 11,
**characterized in that**
the rinsing opening (19) is provided at least area-wise in the resection surface (12).

## Revendications

1. Instrument de résection (2), en particulier pour l'ablation sous-muqueuse, sous-anodermique minimalement invasive de tissus hémorroïdaux (36), comprenant un dispositif porteur oblong effilé (15), lequel est équipé, sur une partie avant, d'un segment de résection (3) pour l'ablation de tissus (36), le segment de résection (3) présentant un renflement en forme d'auge convexe, en forme de cuiller vu en coupe longitudinale, **caractérisé en ce qu'**une surface de résection (12), équipée d'un dispositif de résection (13) fragmentant les tissus, est disposée sur la face interne (7) du renflement en forme d'auge (6), et **en ce qu'**au moins un orifice d'aspiration (18), pouvant être raccordé à une source de dépression (27), est prévu dans la zone de la surface de résection (12).

2. Instrument selon la revendication 1, **caractérisé en ce que** le dispositif porteur (40) présente un segment de préhension (4) par exemple en forme de tige s'étendant dans la direction longitudinale (5), et **en ce que** le renflement en forme d'auge (6) est réalisé, en s'écartant de la direction longitudinale (5), de manière à former un recourbement convexe au moins en partie vers la direction longitudinale (5).

3. Instrument selon la revendication 2, **caractérisé en ce que** le renflement (6), en partant du segment de préhension (4), présente une première courbure externe (40) s'écartant de la direction longitudinale (5) et, contiguë à celle-ci, une deuxième courbure (41) dans le prolongement de la première courbure (40), sur la face interne (7) de laquelle, faisant face à la direction longitudinale (5), est disposée au moins une partie de la surface de résection (12).

4. Instrument selon la revendication 3, **caractérisé en ce que** la surface de résection (12) est exclusivement limitée à la zone de la deuxième courbure.

5. Instrument selon l'une quelconque des revendications précédentes, **caractérisé en ce que** le segment de résection (3) est plus large que le segment de préhension (4) au moins dans la zone du renflement (6).

6. Instrument selon l'une quelconque des revendications précédentes, **caractérisé en ce que** le renflement (6) est réalisé de manière aplatie en coupe transversale par rapport à un segment de préhension (4) du dispositif porteur (40).

7. Instrument selon l'une quelconque des revendications précédentes, **caractérisé en ce qu'**un dispositif d'éclairage (14) est prévu au niveau de la face arrière (16) du segment de résection (3) opposée à la face interne (7).

8. Instrument selon l'une quelconque des revendications précédentes, **caractérisé en ce qu'**un dispositif d'éclairage (14) est disposé au niveau d'une extrémité frontale (11) du segment de résection (3).

9. Instrument selon l'une quelconque des revendications précédentes, **caractérisé en ce qu'**au moins l'extrémité frontale (11) du segment de résection (3) présente un dispositif à ultrasons (17) pour préparer et fragmenter des tissus (36).

10. Instrument selon l'une quelconque des revendications précédentes, **caractérisé en ce que** le segment de résection (3) est réalisé sous la forme d'un dispositif vibrant à ultrasons de fragmentation de tissus (17) pour préparer et fragmenter des tissus (36).

11. Instrument selon l'une quelconque des revendications précédentes, **caractérisé en ce que** le segment de résection (3) présente un orifice de rinçage (19) qui débouche dans un canal de rinçage pouvant être raccordé un système de rinçage.

12. Instrument selon la revendication 11, **caractérisé en ce que**, l'orifice de rinçage (19) est prévu au moins localement dans la surface de résection (12).
